(19) Europäisches Patentamt
European Patent Office
Office européen des brevets

(11) **EP 3 462 575 A1**

(12) **EUROPEAN PATENT APPLICATION**

(43) Date of publication:
**03.04.2019 Bulletin 2019/14**

(51) Int Cl.:
*H02J 50/40* (2016.01)   *H01L 41/04* (2006.01)
*H01L 41/193* (2006.01)   *H02J 50/12* (2016.01)

(21) Application number: **17194012.5**

(22) Date of filing: **29.09.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **HENDRIKS, Cornelis Petrus**
**5656 AE Eindhoven (NL)**

• **HILGERS, Achim**
**5656 AE Eindhoven (NL)**
• **JOHNSON, Mark Thomas**
**5656 AE Eindhoven (NL)**
• **VAN DEN ENDE, Daan Anton**
**5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(54) **WIRELESSLY CONTROLLABLE DEVICE AND SYSTEM AND WIRELESS CONTROL METHOD**

(57)    A wirelessly controllable device (20) comprises responsive material actuator elements (22), each driven by a respective receiver circuit (24) having an inductive coil (26) for receiving electrical energy inductively. Each circuit has a different respective resonance frequency. This allows for wireless selective activation of the actuators by applying a magnetic field having frequency components matching the circuits of only those actuator elements which are desired to be activated. A system (30) is further provided, comprising a wirelessly controllable device and a control unit (28), the control unit having a transmitting inductive coil arrangement (32) for supplying electrical energy to circuits of the wireless device by inductive coupling. A frequency spectrum of the applied field is configured to control which of the actuator elements are activated.

FIG. 3

EP 3 462 575 A1

## Description

FIELD OF THE INVENTION

This invention relates to a device comprising responsive material actuators and configured for wireless control.

BACKGROUND OF THE INVENTION

Responsive materials are a class of materials having properties of reversibly deforming in response to suitable electrically controllable stimulation. Varieties of responsive materials include, by way of example, heat-responsive shape-memory materials, such as shape-memory alloys and shape-memory polymers (stimulated for instance by joule heating), magnetostrictive materials, magnetic shape memory alloys, piezoelectric materials, and photoresponsive materials (photomechanical materials). A further emerging class of materials within the field of responsive materials is that of electroactive materials (EAMs) and electroactive polymers (EAPs) in particular. EAMs can work as sensors or actuators and can be easily manufactured into various shapes allowing easy integration into a large variety of systems.

Controlling responsive material actuators remotely is of use in a range of applications. However, in known solutions, a local signal receiver module is provided wired to the actuators, to receive and decode wirelessly transmitted control signals and to co-ordinate the corresponding control of the actuators. However, use of such modules is not ideal. Multiple wires must be provided to connect the receiver module to the actuators. This introduces complexity and compromises in mechanical and electrical reliability (since any faulty connections may lead to breakdown in operation). Furthermore, the receiver module consumes space. In applications where small form factor is important, this may be a significant difficulty.

One area in particular where the above factors are highly significant is that of implantable devices. Responsive materials, in particular electroactive polymers, enable soft, silent and low power sensors and actuators in a small form factor, which is ideal for use in implantable devices. For these devices, it is highly valuable to enable wireless controllability of the implantable device, to avoid the need for skin-penetrating wires.

For an implantable device, small form factor is clearly highly significant, as is mechanical and electrical reliability, to avoid the need to remove an implanted device to repair faulty connections or control electronics.

There is a need therefore for a means of wirelessly controlling multiple responsive material actuators in a manner that does not require a local signal receiver module or associated connecting electronics.

SUMMARY OF THE INVENTION

This need is at least partly fulfilled with a device and method as defined by the independent claims.

The actuator elements, each comprise a responsive material which can deform in response to an electrical stimulus provided to the actuator element such that the deformation causes the activation of the actuator element. Such actuation can be in the form of strain and/or stroke output such that electrical energy (power) is transformed in mechanical energy (power) by the actuator element upon its activation.

There is thus provided a device having actuator elements configured for wireless actuation through a mechanism of selective resonant inductive coupling. In particular, each actuator element is coupled to a respective circuit tuned to a respective resonance frequency, and comprising an inductive receiver coil. The receiver coil is configured to receive energy through inductive coupling. By tuning each receiver circuit to a different resonant frequency, the actuator elements may be selectively addressed through applying a magnetic field with suitably tuned frequency components.

In general, a resonant circuit tuned to a given frequency will only inductively couple with sufficient efficiency to a supplied field where the frequency spectrum of that field contains a component which matches that resonance frequency. Only in this case is the actuator element supplied by that circuit then activated. If the field does not contain a suitably matched frequency component, the given actuator element remains inactive. In this way, the actuators of the device may be suitably selectively activated through application of a magnetic field being suitably frequency matched.

The resonance frequency of each receiver circuit is tuned to a different respective frequency. The tuning may typically be done in advance, e.g. during fabrication of the device. The tuning typically consists simply of selecting a capacitor and inductor having suitable capacitance and inductance to realize the desired frequency.

The resonance frequency of each receiver circuit is a function of both an inductance of the inductive receiver coil and a capacitance of the capacitor. The resonance frequency of each circuit may be configured either through varying the capacitance of the capacitor, by varying the inductance of the inductive coil, or through a combination of both.

Each actuator element may in examples comprise an actuator member comprising the responsive material and an electrode arrangement arranged for electrically stimulating the actuator member.

The actuator may for example comprise a body of responsive material, either alone, or in combination with one or more other structural elements or components. The responsive material body may be coupled to a backing layer for instance, for inducing bending action.

The actuator element may comprise a layer structure including one or more active layers comprising responsive material, and optionally one or more passive layers, for instance including a backing layer.

The actuator element may include, in examples, a body comprising a passive matrix material having responsive material elements embedded therein.

In accordance with a particular set of embodiments, the wirelessly controllable device may be an implantable device.

By implantable device is meant a device for implantation in the human or animal body, for instance a medically implantable device. Preferred examples are devices for implantation in a human body.

The device may for example be or include a blood pressure sensor (Cardiomems), a restenosis sensor (e.g. iStent), or device for facilitating controlled drug delivery, such as for instance a micro-peristaltic pump (MPS microsystems).

In alternative examples, the device may not be an implantable device. Embodiments of the invention are ideal for use in any application in which relatively short range wireless control (and wireless power supply) of an actuator-containing device may be useful. Any application where permanent local supply of power is difficult or impossible or wireless control with minimal local control electronic electronics is desired could value from application of embodiments of the present invention.

In accordance with one set of embodiments, the responsive material may be an electroactive material, and preferably an electroactive polymer. Electroactive materials (EAMs) are an emerging class of responsive material, and electroactive polymers are one particularly advantageous class of electroactive material. EAMs can work as sensors or actuators and can be easily manufactured into various shapes allowing easy integration into a large variety of systems. EAMs will be discussed in greater detail below. EAPs in particular possess many advantages which may render them superior to other forms of responsive material in embodiments of the invention. These advantages will be discussed in the next section.

Examples in accordance with a further aspect of the invention provide a system, comprising: a wirelessly controllable device in accordance with any embodiment or example described above or below or as defined in any claim of the present application; and a control unit, the control unit comprising: a transmitter coil arrangement operable to generate a magnetic field for supplying electrical energy to the receiver circuits of the wirelessly controllable device through inductive coupling, and a controller, operatively coupled with the transmitter coil arrangement, and adapted to facilitate selective activation of the actuator elements through controlling a frequency spectrum of the magnetic field to include frequency components selectively matching selected of said resonance frequencies of the receiver circuits.

A system is thus provided in accordance with the present aspect of the invention which includes both a wirelessly controllable device as described above and a control unit for selectively activating different of said actuator elements by applying an oscillatory magnetic field of suitably matched frequency to the receiver circuit resonance frequencies.

A transmitter coil arrangement is provided within the control unit of the system. The transmitter coil arrangement includes at least one inductive coil for generating a magnetic field, and for inductively coupling with the inductive coils of the plurality of receiver circuits of the wirelessly controllable device, in order to selectively supply them with power. Only those circuits with resonance frequencies matching or substantially matching frequency components of the magnetic field generated by the transmitter coil arrangement will be supplied with sufficient electrical power to activate their corresponding actuator element. Hence, by controlling the frequency spectrum of the field, it can be controlled which actuators are activated.

This solution also avoids the need to provide a local power supply within the wirelessly controllable device for powering the actuator elements. All power is instead provided inductively by the control unit. The control unit may have a local dedicated power supply (for instance a battery) or may have a connection for connecting with a mains supply.

In accordance with one or more examples, the controller may be adapted to control the transmitter coil arrangement to generate a magnetic field having a single frequency component which varies over time between different of the resonance frequencies to thereby realize sequential activation of different of the actuator elements.

In accordance with further embodiments, the controller may be adapted to control the transmitter coil arrangement to generate a field comprising multiple superposed frequency components matching different of the resonance frequencies, to thereby realize simultaneous activation of different of the actuator elements. This may be achieved in examples with a single inductive transmitter coil controlled to generate a field having a field vs. time function being the superposition of field-time functions of multiple individual frequencies. The resultant field might be calculated in advance by the control unit, e.g. through suitable analog electronics or through a suitable processor, and the coil the controlled to produce the calculated field function.

In accordance with one or more sets of embodiments, the transmitter coil arrangement may comprise a plurality of individual inductive transmitter coils, each controlled by the controller to generate a respective magnetic field of a different respective frequency, each matching a resonance frequency of a respective one of the receiver circuits.

The multiple coils may be controlled non-simultaneously, to realize for instance sequential activation of the actuator elements.

Alternatively, the coils may be controlled to generate said respective fields simultaneously to thereby realize simultaneous activation of a plurality of the actuator elements. In this way simultaneous activation of multiple actuator elements may be achieved without the added processing complexity of generating a superposed signal using a single coil. However, this may come at the cost of increased form factor of the control unit.

In accordance with the present set of embodiments, the plurality of coils may be arranged in planar formation relative to one another. This provides an efficient and convenient spatial arrangement for electrically supplying the receiver circuits of the wirelessly controllable device. In particular, in the case of an implantable device, this allows optimal alignment with the skin surface, and hence optimal alignment of the transmitting coils and the receiving inductive coils for optimal inductive coupling and thus energy transfer.

In accordance with one or more examples, the controller may be adapted to control the transmitter coil arrangement to generate a field comprising a recurring pattern of pulsed wave-packets, each wave-packet comprising two or more superposed frequency components matching resonance frequencies of two or more of the receiver circuits. Here, a magnetic driving signal is generated comprising wave-packets of two or more frequencies, superposed. Preferably the wave-packets are short bursts (for instance milliseconds), and generated recurrently or cyclically such that the responsive material of the actuator elements does not have sufficient time after being first stimulated by one of the bursts to relax to its original (non-deformed) state before the next burst is received. In this manner multiple actuator elements may be activated simultaneously.

In accordance with one or more sets of embodiments, the controller may be adapted to control an amplitude of the displacement of the actuator elements (i.e. the degree or extent of actuation or the actuation displacement), through various modifications of the applied magnetic field.

In accordance with one set of embodiments, the controller may be adapted to control an amplitude of one or more of the frequency components of the generated field, to thereby control an actuation displacement of the corresponding activated actuator element.

In particular, the actuation displacement of the actuator element coupled to the field whose resonance frequency matches said frequency component is controlled. Since the amplitude of the supplied field is correlated with the supplied power, by varying the amplitude of the field oscillations, the supplied power is varied and hence the degree of actuation of the actuator element. For instance the voltage or current supplied across the responsive material is reduced, thereby reducing the degree to which the responsive material is stimulated to deform.

In further embodiments, the controller may be adapted to control a degree of actuation displacement of a selected actuator element by controlling a degree of correspondence between a frequency component of the magnetic field and the resonance frequency of the circuit supplying the actuator element.

In particular, where a frequency component of the field exactly matches a resonance frequency of a receiver circuit, the actuator element connected to that circuit will be actuated with the maximal power possible given the relative spatial positioning of the respective coils and the amplitude of the supplied frequency. However, it is not necessary that the frequency be precisely matched in order for an actuator element to deform to some extent. Where a frequency component of an applied field at least substantially matches the resonance frequency of a receiver circuit, some actuation will take place. By deliberately varying the degree to which a frequency component of the field matches the resonant frequency of a circuit, the quality of resonant inductive coupling can be varied, and hence the power supplied can be varied.

In accordance with one or more sets of embodiments, the controller may be adapted to control the coil to generate a pulsed field, and wherein the controller is adapted to vary an amplitude displacement of one or more activated actuator elements by varying a duty cycle of at least corresponding matched frequency components of the field.

This set of embodiments is based on varying supplied electrical power through duty cycle modulation techniques. By simply reducing the duty cycle, supplied power can be reduced and hence actuation displacement reduced, and vice versa.

Examples in accordance with a further aspect of the invention provide a method of wirelessly controlling a device, the device comprising: a plurality of actuator elements, each comprising a responsive material, deformable in response to an electrical stimulus, each actuator element being electrically connected with a respective receiver circuit for electrically supplying the element for supplying electrical stimuli for activating the element, the circuit including an inductive coil for receiving electrical energy by inductive coupling, and a capacitor for tuning, in combination with the inductive coil, a resonance frequency of the circuit, and wherein each receiver circuit is tuned with a different resonance frequency,

and the method comprising applying a magnetic field to the device have a frequency spectrum controlled to include frequency components selectively matching one or more of the resonant frequencies of the receiver circuits to thereby effect selective activation of a corresponding one or more of the actuator elements.

BRIEF DESCRIPTION OF THE DRAWINGS

Examples of the invention will now be described in detail with reference to the accompanying drawings, in which:

> Figs. 1 and 2 show two possible operating modes for an EAP device;
> Fig. 3 schematically depicts an example system in accordance with one or more embodiments of the invention, the system comprising an example wirelessly controllable device in accordance with one or more embodiments of the invention;

Fig. 4 illustrates a frequency vs. time progression of an example magnetic field applied to control an example wirelessly controllable device in accordance with one or more embodiments of the present invention;

Fig. 5 schematically depicts a further example wirelessly controllable device in accordance with one or more embodiments; and

Fig. 6 schematically depicts a further example wirelessly controllable device in accordance with one or more embodiments.

DETAILED DESCRIPTION OF THE EMBODIMENTS

The invention provides a wirelessly controllable device comprising responsive material actuator elements, each driven by a respective receiver circuit having an inductive coil for receiving electrical energy inductively. Each circuit has a different respective resonance frequency. This allows for wireless selective activation of the actuators by applying a magnetic field having frequency components matching the circuits of only those actuator elements which are desired to be activated. A system is further provided comprising such a wirelessly controllable device and a control unit, the control unit having a transmitting inductive coil arrangement for supplying electrical energy to circuits of the wirelessly controllable device by inductive coupling. A frequency spectrum of the applied field is configured to control which of the actuator elements are activated.

Embodiments of the invention make use of responsive materials to provide actuator elements.

In preferred embodiments of the invention, the responsive material elements comprise electroactive material (EAM). As noted briefly above, EAMs are a class of materials within the field of electrically responsive materials. When implemented in an actuation device, subjecting an EAM to an electrical drive signal can make them change in size and/or shape. This effect can be used for actuation and sensing purposes. There exist inorganic and organic EAMs. One particular kind of organic EAM is that of electroactive polymers (EAPs).

Electroactive polymers (EAPs) are an emerging class of materials within the field of electrically responsive materials. EAPs can work as sensors or actuators and can easily be manufactured into various shapes allowing easy integration into a large variety of systems.

Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation.

The improved performance and particular advantages of EAP material give rise to applicability to new applications.

An EAP device can be used in any application in which a small amount of movement of a component or feature is desired, based on electric actuation. Similarly, the technology can be used for sensing small movements.

The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 1MHz, most typically below 20 kHz.

Devices using electroactive polymers can be subdivided into field-driven and ionic-driven materials.

Examples of field-driven EAPs include Piezoelectric polymers, Electrostrictive polymers (such as PVDF based relaxor polymers) and Dielectric Elastomers. Other examples include Electrostrictive Graft polymers, Electrostrictive paper, Electrets, Electroviscoelastic Elastomers and Liquid Crystal Elastomers.

Examples of ionic-driven EAPs are conjugated/conducting polymers, Ionic Polymer Metal Composites (IPMC) and carbon nanotubes (CNTs). Other examples include ionic polymer gels.

Field-driven EAPs are actuated by an electric field through direct electromechanical coupling. They usually require high fields (tens of megavolts per meter) but low currents. Polymer layers are usually thin to keep the driving voltage as low as possible.

Ionic EAPs are activated by an electrically induced transport of ions and/or solvent. They usually require low voltages but high currents. They require a liquid/gel electrolyte medium (although some material systems can also operate using solid electrolytes).

Both classes of EAP have multiple family members, each having their own advantages and disadvantages.

A first notable subclass of field driven EAPs are Piezoelectric and Electrostrictive polymers. While the electromechanical performance of traditional piezoelectric polymers is limited, a breakthrough in improving this performance has led to PVDF relaxor polymers, which show spontaneous electric polarization (field driven alignment). These materials can be pre-strained for improved performance in the strained direction (pre-strain leads to better molecular alignment). Normally, metal electrodes are used since strains usually are in the moderate regime (1-5%). Other types of electrodes (such as conducting polymers, carbon black based oils, gels or elastomers, etc.) can also be used. The electrodes can be continuous, or segmented.

Another subclass of interest of field driven EAPs is that of Dielectric Elastomers. A thin film of this material may be sandwiched between compliant electrodes, forming a parallel plate capacitor. In the case of dielectric elastomers, the Maxwell stress induced by the applied electric field results in a stress on the film, causing it to contract in thickness and expand in area. Strain performance is typically enlarged by pre-straining the elastomer (requiring a frame to hold the pre-strain). Strains can be considerable (10-300%). This also constrains the type of electrodes that can be used: for low and moderate strains, metal electrodes and conducting polymer electrodes can be considered, for the high-strain regime, carbon black based oils, gels or elastomers are typically used. The electrodes can be continuous, or segmented.

A first notable subclass of ionic EAPs is Ionic Polymer Metal Composites (IPMCs). IPMCs consist of a solvent swollen ion-exchange polymer membrane laminated between two thin metal or carbon based electrodes and requires the use of an electrolyte. Typical electrode materials are Pt, Gd, CNTs, CPs, Pd. Typical electrolytes are Li+ and Na+ water-based solutions. When a field is applied, cations typically travel to the cathode side together with water. This leads to reorganization of hydrophilic clusters and to polymer expansion. Strain in the cathode area leads to stress in rest of the polymer matrix resulting in bending towards the anode. Reversing the applied voltage inverts bending. Well known polymer membranes are Nafion® and Flemion®.

Another notable subclass of Ionic polymers is conjugated/conducting polymers. A conjugated polymer actuator typically consists of an electrolyte sandwiched by two layers of the conjugated polymer. The electrolyte is used to change oxidation state. When a potential is applied to the polymer through the electrolyte, electrons are added to or removed from the polymer, driving oxidation and reduction. Reduction results in contraction, oxidation in expansion.

In some cases, thin film electrodes are added when the polymer itself lacks sufficient conductivity (dimension-wise). The electrolyte can be a liquid, a gel or a solid material (i.e. complex of high molecular weight polymers and metal salts). Most common conjugated polymers are polypyrrole (PPy), Polyaniline (PANi) and polythiophene (PTh).

An actuator may also be formed of carbon nanotubes (CNTs), suspended in an electrolyte. The electrolyte forms a double layer with the nanotubes, allowing injection of charges. This double-layer charge injection is considered as the primary mechanism in CNT actuators. The CNT acts as an electrode capacitor with charge injected into the CNT, which is then balanced by an electrical double-layer formed by movement of electrolytes to the CNT surface. Changing the charge on the carbon atoms results in changes of C-C bond length. As a result, expansion and contraction of single CNT can be observed.

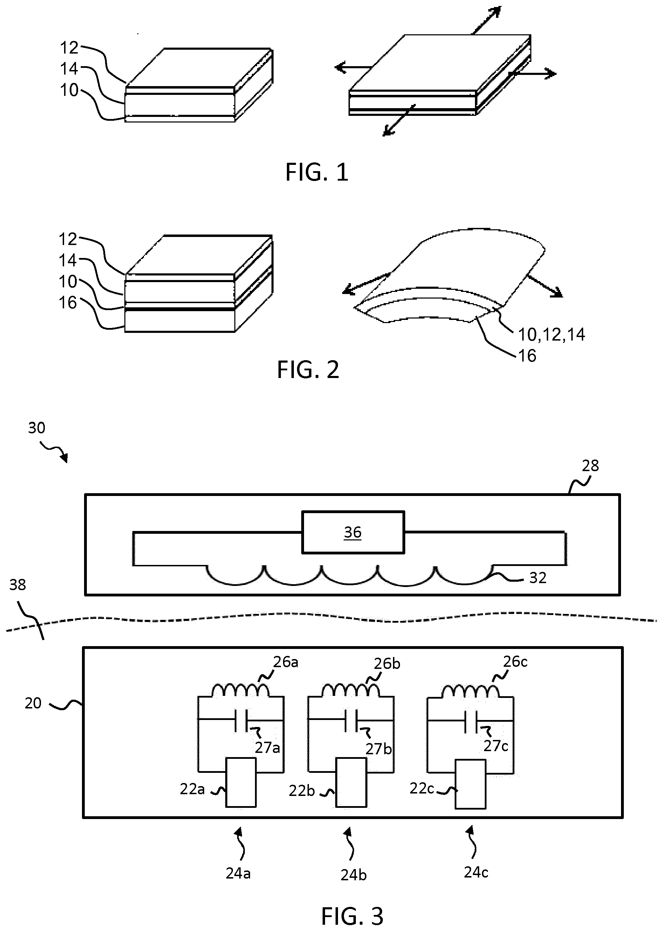

Figs. 1 and 2 show two possible operating modes for an EAP device.

The device comprises an electroactive polymer layer 14 sandwiched between electrodes 10, 12 on opposite sides of the electroactive polymer layer 14.

Fig. 1 shows a device which is not clamped. A voltage is used to cause the electroactive polymer layer to expand in all directions as shown.

Fig. 2 shows a device which is designed so that the expansion arises only in one direction. The device is supported by a carrier layer 16. A voltage is used to cause the electroactive polymer layer to curve or bow.

Together, the electrodes, electroactive polymer layer, and carrier may be considered to constitute the overall electroactive polymer structure.

The nature of this movement for example arises from the interaction between the active layer which expands when actuated, and the passive carrier layer. To obtain the asymmetric curving around an axis as shown, molecular orientation (film stretching) may for example be applied, forcing the movement in one direction.

The expansion in one direction may result from the asymmetry in the EAP polymer, or it may result from asymmetry in the properties of the carrier layer, or a combination of both.

An electroactive polymer structure as described above may be used both for actuation and for sensing. The most prominent sensing mechanisms are based on force measurements and strain detection. Dielectric elastomers, for example, can be easily stretched by an external force. By putting a low voltage on the sensor, the strain can be measured as a function of voltage (the voltage is a function of the area).

Another way of sensing with field driven systems is measuring the capacitance-change directly or measuring changes in electrode resistance as a function of strain.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured. It has also been shown that the resistance of CNTs change when in contact with gaseous molecules (e.g. $O_2$, $NO_2$), making CNTs usable as gas detectors.

One application area of particular interest in the context of the present invention is that of implantable devices.

In particular, there exists an unmet clinical need for accurate, unobtrusive and long term monitoring of patients with for instance chronic diseases such as heart failure, peripheral artery disease or hypertension. Wearable devices or skin-insertable devices do not sufficiently serve this need because their relatively peripheral positioning does not allow them direct access to the cardiovascular system.

To provide these functions, 'smart' implantable devices are increasingly being considered necessary. In general, "smart" in this context implies the integration into the devices of one or more sensors and actuators. These may include for instance blood pressure sensors (Cardiomems), restenosis sensors (iStent), or actuators for controlled drug delivery, such as micro-peristaltic pumps (MPS microsystems). With regards more active functionality, envisioned functions include for example blood flow restriction, stent delivery, acoustic antifouling, and controlled drug delivery.

Responsive materials, in particular electroactive polymers, enable soft, silent and low power sensors and actuators in a small form factor. This hence makes them ideal for implementation within implantable devices which typically require small form factor, low power (for safety and practicality) and silent operation.

The principles of invention will be described with reference to Fig. 3, which shows a system 30 in accordance with an aspect of the invention, the system including a wirelessly controllable device 20, said device being in accordance with a further aspect of the invention, and a control unit 28. The example system and device shown are in the form of an implantable device, shown implanted within an area of bodily tissue 38. However, this is purely by way of illustration, and it will naturally be understood by the skilled person that this illustrated device as system may be implemented without loss of functionality in any other context or application in which the wirelessly controllable device is not implanted in tissue.

Fig. 3 schematically depicts an example system 30 in accordance with one or more embodiments of one aspect of the invention. The system comprises a remotely controllable device 20, the device being in accordance with an embodiment of a further separate aspect of the invention. The system further comprises a control unit 28 for remotely controlling actuation of, and providing electrical power to, actuator elements 22 of the remotely controllable device.

The remotely controllable device comprises a plurality of actuator elements 22a, 22b, 22c, each actuator element being electrically connected with a respective receiver circuit 24a, 24b, 24c for electrically supplying the actuator element. Each of the receiver circuits comprises a respective inductive coil 26a, 26b, 26c for receiving electrical energy (from the control unit 28) by inductive coupling, and a respective capacitor 27a, 27b, 27c, connected in parallel with the inductive coil. Although in the particular example of Fig. 3, three receiver circuits and actuator elements are shown in particular, this is by way of example only; other examples may comprise any plural number of actuator elements and respective receiver circuits.

Each of the actuator elements 22 is electrically coupled to a respective receiver circuit 24 for electrically supplying or driving the respective actuator element. Each receiver circuit comprises an inductive coil 26 for receiving electrical energy (e.g. from the control unit 28) through inductive coupling. Each of the receiver circuits further comprises a capacitor 27.

The inductance of the inductive coil 26 and capacitance of the capacitor 27 are selected for each receiver circuit such that each receiver circuit is 'tuned' to have a different characteristic resonant frequency. This enables selective supply of energy to the receiver circuits, and hence selective activation of the actuator elements 22 through selective resonant inductive coupling, wherein an applied magnetic field is controlled to comprise frequency components which match the resonant frequencies of only those circuits whose actuator elements are desired to be activated.

Each circuit may, in examples, be tuned to a desired resonant frequency based upon the standard expression for a resonant LC circuit $\omega_0 = \frac{1}{\sqrt{LC}}$, where $\omega_0$ gives the resonant angular frequency (in units of radians per second) of the circuit and where L is the total inductance of the receiver circuit (in units of Henrys) and C is the total capacitance of the receiver circuit (in units of Farads). For a simple circuit comprising a single inductive coil and single capacitor, the capacitance C of the circuit may be approximated to be equal to the capacitance of the capacitor 27 of the circuit, and the inductance L of the circuit may be approximated to be equal to the inductance of the inductive coil 26 of the circuit.

The same expression may be represented in in the alternate form $f_0 = \frac{1}{2\pi\sqrt{LC}}$ where $f_0$ is the resonant frequency in units of Hertz.

A required capacitance or inductance for a circuit which is to be tuned to a desired frequency may therefore be determined simply by rearranging either of the expressions provided above. Where either the inductance or capacitance of the circuit is fixed or known in advance, the expression may be re-arranged to provide the required capacitance or inductance respectively for the circuit in order to achieve the desired resonance frequency. Hence, either both the capacitance of the capacitor 27 and the inductance of the inductor 26 may be selected for each circuit in order to tune the circuit to a given resonant frequency, or more simply, each circuit may have a fixed inductance (or capacitance) and simply the capacitance of the capacitor alone (or, alternatively, the inductance of the inductor alone) may be varied between different circuits. Varying only the capacitance of the capacitor may be preferable because capacitors of different capacitance may be cheaper and simpler to provide.

The actuator elements 22 in the example of Fig. 3 each comprise a responsive material deformable in response to an electrical stimulus. For the example of Fig. 3, each actuator element is assumed to comprise an electroactive material, where in particular this may be an electroactive polymer material. However, this is by way of example only. In further examples, the actuator element may comprise a different responsive material, for instance a smart memory alloy, deformable by thermal stimulation, induced for instance by joule heating of the actuator element 22 through application of an electrical current.

Each of the actuator elements 22 may comprise an actuator member comprising the responsive material and an electrode arrangement arranged for electrically stimulating the actuator member. The actuator member may for instance be a body consisting of the responsive material, or may be a body comprising one or more responsive material components, for instance one or more responsive material layers or a plurality of responsive material particles. The actuator member may comprise a layer stack of responsive material layers, either all of the same responsive material or of different responsive materials.

The electrode arrangement may comprise a single pair of electrodes disposed surrounding or sandwiching the actuator member for generating an electric field across the actuator member. Alternatively, the electrode arrangement may comprise more than two electrodes, where these may be interposed within the body of the actuator member, for instance between layers of the member (where the member comprises layers) for stimulating individual layers.

Although in the particular example of Fig. 3, a dedicated capacitor 27 provided within each receiver circuit, in accordance with one or more alternative examples, the actuator element 22 itself may be configured to function as a capacitor within a given receiver circuit. Responsive material bodies, e.g. EAP bodies, exhibit a capacitance when a field or current is applied across them. By appropriately selecting or configuring the intrinsic capacitance of the actuator element, the resonant frequency of the circuit may be configured in the same manner as where a separate capacitor is provided.

Above has been described the wirelessly controllable device 20 of Fig. 3, adapted to enable wireless individualized control of a plurality of actuator members comprised by the device.

The system 30 shown in Fig. 3 further comprises a control unit 28 for controlling the wirelessly controllable device 20.

The control unit 28 comprises a transmitter coil arrangement 32 operable to generate a magnetic field for supplying electrical energy to the receiver circuits 24 of the wirelessly controllable device 20 through inductive coupling.

A controller 36 is operatively coupled with the transmitter coil arrangement 32, for controlling generation by means of the coil arrangement of an oscillatory magnetic field. The controller is adapted to effect selective activation of the actuator elements 22 by controlling a frequency spectrum of the magnetic field to include frequency components selectively matching selected of said resonance frequencies of the receiver circuits. The transmitter coil arrangement comprises one or more inductive coils for supplying electrical energy to the receiver circuits 24 via inductive coupling. By an 'oscillatory' or 'oscillating' magnetic field is meant an alternating or sinusoidal field.

As will be understood by the skilled person, creating an oscillating magnetic field using an inductive coil may consist in simply applying an alternating current through the coil at an AC frequency which matches the desired frequency, or desired frequency vs. time function, of the magnetic field to be generated.

In accordance with a first example, the controller 36 may be adapted to control the transmitter coil arrangement 32 to generate a magnetic field having a single frequency component which varies over time between different of the resonance frequencies $f_1$, $f_2$, $f_3$ of the receiver circuits, to thereby realize sequential activation of different of the actuator elements. The transmitter coil arrangement may in this example comprise just a single inductive coil, wherein the AC frequency of the current applied through the coil is varied as a function of time to thereby realize the oscillatory magnetic field of corresponding time-varying frequency. Alternatively, the transmitter coil arrangement may comprise a plurality of coils, each configured for or controlled to generate a field of a fixed respective frequency, and wherein these coils are controlled to activate sequentially in turn in order thereby to realize the time-varying magnetic field applied to the receiver coils and the sequential activation of the actuator elements.

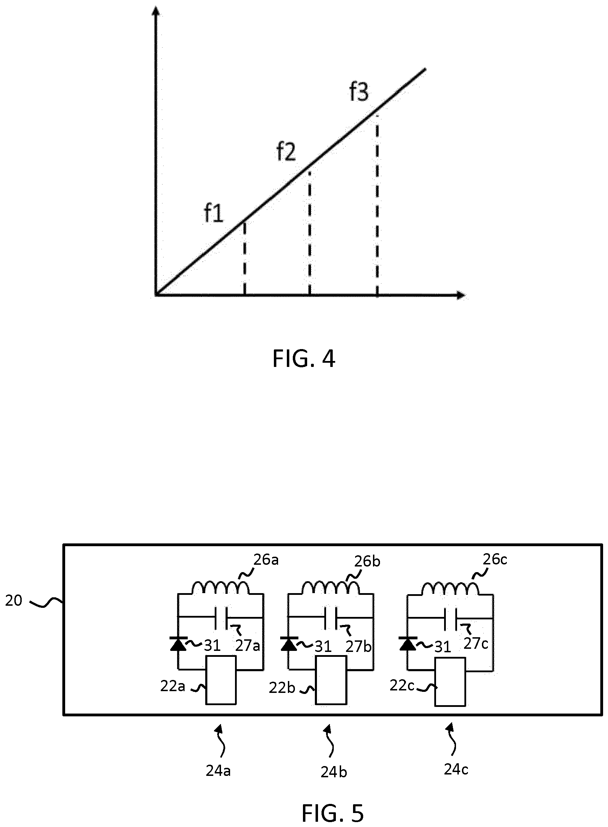

Fig. 4 schematically illustrates the frequency progression as a function of time of the (oscillatory) magnetic field which may be applied in accordance with this example. The frequency of the applied field is shown on the y-axis (arbitrary units), and the time on the x-axis (arbitrary units). As shown, the field frequency varies (linearly, in the illustrated example) as a function of time, moving progressively between a resonant frequency, $f_1$, of a first of the receiver frequencies, to a resonant frequency, $f_2$, of a second, to a resonant frequency $f_3$ of the third. When the applied field is oscillating at the resonant frequency of a respective receiver circuit, the actuator element of that circuit is activated; when the field is oscillating at a frequency substantially different to the resonant frequency of the respective receiver circuit, the actuator element of that circuit is deactivated.

It can be seen therefore that by applying the exemplary field illustrated by Fig. 4, the field is controlled to sweep through the respective resonant frequencies of each of the receiver circuits 24, and thereby effect sequential activation of each of the actuator elements 22a, 22b, 22c in turn.

In accordance with a further set of examples, the controller 36 may be adapted to control the transmitter coil arrangement to generate a field comprising multiple superposed frequency components matching different of the resonance frequencies, to thereby realize simultaneous activation of different of the actuator elements. The applied oscillatory field in this case has a frequency spectrum which comprises frequency components which match or substantially match resonant frequencies of two or more of the receiver circuits 24. In this way, two or more of the actuator elements may be activated simultaneously.

In accordance with this set of examples, the transmitter coil arrangement may comprise a plurality of individual (inductive) transmitter coils, each controlled by the controller to generate a respective magnetic field of a different respective frequency, matching a resonance frequency of a respective one of the receiver circuits. In this way, a composite field comprising multiple frequency components may be straightforwardly generated, by controlling the coils to generate said respective fields simultaneously.

The multiple transmitter coils may be stacked such that a flat (i.e. substantially planar) form factor is achieved. Preferably, the multiple transmitter coils may be arranged within the control unit 28 such that, in use, upon holding the control unit above the remotely controllable device (e.g. as illustrated in Fig. 3), the coils are aligned with the receiver coils 26 of the receiver circuits. For example, the spacing between the receiver coils 26 may be the same as the spacing between multiple transmitter coils of the transmitter coil arrangement 32.

Alternatively to providing a transmitter coil arrangement comprising multiple transmitter coils, a single coil may be provided, and wherein the single coil is controlled by the controller 36 to generate an oscillatory field having a frequency spectrum which includes multiple frequency components, each matching or substantially matching a respective resonant frequency of one of the receiver coils 26 of the receiver circuits 22. This may reduce the form factor of the control unit 28 since fewer coils need to be incorporated, but at the potential cost of slightly more complex control electronics within the controller 36 in order to effect the multiple-frequency-component magnetic field.

There may for example be provided electronics within the controller 36 adapted to calculate or determine a field vs. time function corresponding to a given superposition of field frequencies, and to control the transmitter coil arrangement to generate this determined field through appropriate application control of a current through the coil as a function of time.

In accordance with one or more further examples, the controller may be adapted to control the transmitter coil arrangement to generate a field comprising a repeating pattern of pulsed wave-packets, each comprising two or more superposed frequency components matching resonance frequencies of two or more of the receiver circuits.

In accordance with these examples, a plurality of the actuator elements 22 may be controlled to activate simultaneously through controlling the transmitter coil arrangement 32 to provide a driving magnetic field comprising wave-packets formed of two or more superposed frequency components, each matching the respective resonant frequency of one of the receiver circuits 24. In preferred cases, the wave-packets may be provided in the form of short 'bursts', each having total duration in the order of milliseconds, with pause time between each burst also in the order of milliseconds. In this way, the responsive material of the actuator element will have no time to relax to its non-stimulated (non-actuated) state before the next burst is applied by the transmitter coil arrangement and applied to the actuator element. In this manner, each of the actuator elements is continually maintained in an active, actuated state.

Typically, the applied RF field frequencies may be in the order of MHz. Accordingly, each burst (having duration in the order of milliseconds) may comprise in the order of thousands of RF cycles. By providing bursts having duration in the order only of milliseconds, possible over-vibration of the actuator element upon application and removal of the power may be mitigated or avoided.

In accordance with these examples, the transmitter coil arrangement may comprise a single transmitter coil or may comprise a plurality of transmitter coils. Where the coil arrangement comprises a single coil, the controller may be adapted to calculate or determine the resultant field vs. time function corresponding to a particular superposition of fields of two or more of the resonant frequencies. The controller may then be adapted to control the transmitter coil to generate pulses of the required short duration, having said determined field pattern.

Alternatively, where the transmitter coil arrangement comprises a plurality of individual transmitter coils, two or more of these may be controlled simultaneously to generate fields of different respective frequencies, thereby generating a resultant field comprised of a superposition of said two or more frequencies. The coils may be activated in a pulsing manner, such as to thereby generate the required short duration pulses.

The above pulsing control approach has the advantage that average applied power is reduced, since power is applied intermittently. Energy consumption is therefore reduced. This may be achieved however without any consequent reduction in actuation amplitude, provided that bursts are of sufficient duration and applied at sufficient frequency.

A further advantage is that pulsed power supply permits control of actuation displacement through varying of the pulse duty cycle. This is described in greater detail below.

There have thus been described above a number of control approaches which may be implemented in accordance with aspects of the present invention in order to effect selective activation of different of the actuator elements 22 of the wirelessly controllable device 20.

In accordance with any embodiment of these aspects, there may further be implemented, in combination with any described embodiment, or any embodiment disclosed or covered by the claims of the present application, means for controlling a degree of actuation of the actuator elements which are selectively activated.

In accordance with a first set of examples, the controller may be adapted to control an amplitude of one or more of the frequency components of the generated magnetic field, to thereby control an actuation displacement of the corresponding selectively activated actuator element. Hence, whether said frequency components of the field are applied simultaneously or sequentially, an amplitude of each frequency component may be individually controlled in accordance with a desired actuation displacement of the corresponding actuator element (i.e. the element whose receiver circuit has a corresponding resonance frequency).

In particular, an actuator element may be controlled to deform with greater displacement amplitude by including a frequency component of greater magnetic field amplitude, and vice versa. The higher field amplitude will generate a higher voltage in the corresponding receiver circuit coupling to that frequency component, and thereby realize a greater actuation displacement of the responsive-material-comprising actuator element.

This may be particularly effective where the responsive material is an electroactive material, since it is known that the applied voltage is directly related to the resultant generated displacement amplitude.

Additionally or alternatively, in accordance with one or more examples, the controller 36 may be adapted to control a degree of actuation displacement of a selectively activated actuator element 22 by controlling a degree of correspondence between a matched frequency component of the generated magnetic field and a resonance frequency of the receiver circuit 24 supplying the actuator element.

The degree of actuation displacement will vary depending upon how precisely matched the frequency of the applied field component is to the resonance frequency of the receiver circuit. Where the field frequency (component) precisely matches the resonance frequency of the circuit, there will be maximal coupling between that field component and the circuit and maximal voltage provided therefore to the actuator element 22 (and so maximal actuation displacement). However, as the frequency of the applied field component moves very slowly away from the resonance frequency of the circuit, so the coupling between the field and the circuit progressively decreases, resulting in a gradually decreasing voltage applied to the actuator element 22 and so a correspondingly decreasing actuation voltage.

By way of example, an applied frequency component may be varied in its frequency within a range of approximately +/- 4-5% of the resonant frequency of the receiver circuit, or alternatively within a range approximately +/- 2-3% or within a range approximately +/- 1-2, depending upon the degree of amplitude modulation desired. The greater the disparity with the resonance frequency, the greater is the reduction in actuation displacement.

Hence, in accordance with this example, a mechanism is employed in which actuation displacement is controlled through controlling a degree of correspondence between frequency components of the generated magnetic field and the resonance frequencies of the receiver circuits. Actuation displacement of a selected actuator element 22 may be reduced by displacing or offsetting by a small amount the frequency of the applied field component as compared with the resonant frequency of the corresponding receiver circuit of that actuator element.

In accordance with a further set of examples, the controller may be adapted to control the transmitter coil to generate a pulsed field, and wherein the controller is adapted to vary an amplitude displacement of one or more activated actuator elements by varying a duty cycle of at least corresponding matched frequency components of the field.

Hence here, the actuator elements are selectively activated through applying field pulses comprising superposed frequency components corresponding to resonance frequencies of the receiver circuits 24. The actuation displacement of the actuator elements may be controlled by varying the duration of each of the applied pulses. The duration of each frequency component within a given superposed pulse can be individually varied, i.e. each frequency component may be applied in pulses of a duration commensurate with a relative desired actuation displacement for a corresponding actuator element 22.

Where a shorter duty cycle is thus used, this provides the responsive material within the corresponding actuator element 22 time to relax back from maximum actuation, thus resulting in time-averaged actuation displacement (that which is perceptible to humans) which is less than the maximum actuation displacement of given the actuator element 22. In this way duty cycle can be used to control actuation displacements of each of the individual actuator elements 22.

In accordance with one or more embodiments, the receiver circuits 24 may include rectifier means for converting applied AC signals to DC signals. The rectifier means regulates the charge transfer to the actuator element 22 by the receiver circuit. This may be of particular advantage for instance where an EAP actuator element is provided.

Fig. 5 shows an example.

As illustrated, a diode 31 is provided within each receiver circuit, connected in series between the actuator element 22 and the capacitor 27 (and inductive coil 26). Although, a simple diode is used in the illustrated example, any other rectifiers may instead be used. A range of possible examples will be apparent to the skilled person.

The configuration of Fig. 5 permits the actuator element 22 to be actuated by fixed (DC) voltage operation, despite the AC source provided by the inductive coil 26 (which supplies a current correspondent with the applied alternating magnetic field). This is useful for instance where a fixed or static actuation of the actuator element 22 is desired, as opposed to a vibrating actuation action. The self-discharge rate of the combination of the actuator element and rectifier means (diode in this case) determines the time taken for full actuator element discharge (i.e. the full cycle time).

In accordance with a further set of one or more embodiments, the each of the receiver circuits 24 may further comprise a resistor connected in parallel with components of the circuit, for regulating cycle time of the actuator element.

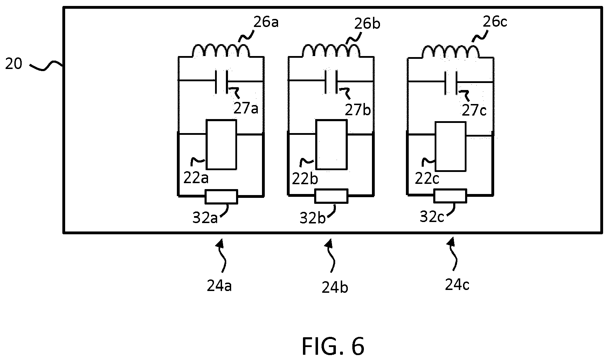

Fig. 6 shows an example.

As illustrated, a resistor 32 is included in each receiver circuit 24 coupled in parallel with all other components. The parallel resistor allows for regulating the discharging speed of the actuator element (which in advantageous examples may be an EAP actuator element). This allows the duty cycle time of the actuator element actuation to be regulated. Low frequency operation of the actuator element may for instance be implemented, wherein the charging speed of the actuator element 22 (governed by the receiver circuit) and the discharging speed (governed by the parallel resistor 32) determine the operating frequency of the actuator.

Above has been presented examples in the application area of implantable devices.

Particular implantable device applications for which the present invention is suitable include:

- use as part of a peristaltic pump for controlled drug delivery;
- shape adaptation of stent grafts after their placement in order to prevent leakage;
- use in tissue-characterizing sensor-actuator arrays;
- use to provide an adaptive heart valve annulus to prevent leakage;
- stents with adaptive restriction which varies over their length (e.g. to adapt pressure or flow rate).

However, the present invention is also applicable to a wide range of other application areas, outside of that of implantable devices. These include by way of example soft robotics applications (meaning robotics applications in which rigid modules such as mechatronic actuators are desired to be avoided, and/or where weight is desired to be minimized). These also include by way of example (soft) microfluidic systems (where again rigid actuation elements may be undesirable), and vacuum systems (e.g. where component parts need to be minimized to reduce contamination).

Any application in which a wirelessly addressable assembly of actuators (over a relatively short range) may be useful, in particular where wireless (i.e. inductive) supply of power may also be useful, may benefit from the device, system and/or method of the present invention.

Although in the detailed description herein above the construction and operation of certain example devices and systems according to the invention have been described as employing EAPs in particular, the invention may in fact be used for devices based on other kinds of responsive material, including other kinds of EAM material. Hence, unless indicated otherwise, the EAP materials hereinabove can be replaced with other responsive materials such as other EAM materials. Other responsive materials include, by way of example, heat-responsive shape-memory materials, such as shape-memory alloys and shape memory polymers (stimulated for instance by joule heating), magnetostrictive materials, magnetic shape memory alloys, piezoelectric materials, and photoresponsive materials (photomechanical materials).

Such other responsive materials are known in the art and the person skilled in the art will know where to find them and how to apply them.

Materials suitable for an EAP element are known. Electro-active polymers include, but are not limited to, the sub-classes: piezoelectric polymers, electromechanical polymers, relaxor ferroelectric polymers, electrostrictive polymers, dielectric elastomers, liquid crystal elastomers, conjugated polymers, Ionic Polymer Metal Composites, ionic gels and polymer gels.

The sub-class electrostrictive polymers includes, but is not limited to:

Polyvinylidene fluoride (PVDF), Polyvinylidene fluoride - trifluoroethylene (PVDF-TrFE), Polyvinylidene fluoride - trifluoroethylene - chlorofluoroethylene (PVDF-TrFE-CFE), Polyvinylidene fluoride - trifluoroethylene - chlorotrifluoroethylene) (PVDF-TrFE-CTFE), Polyvinylidene fluoride- hexafluoropropylene (PVDF - HFP), polyurethanes or blends thereof.

The sub-class dielectric elastomers includes, but is not limited to: acrylates, polyurethanes, silicones.

The sub-class conjugated polymers includes, but is not limited to: polypyrrole, poly-3, 4-ethylenedioxythiophene, poly(p-phenylene sulfide), polyanilines.

In all of these examples, additional passive layers may be provided for influencing the electrical and/or mechanical behavior of the EAP element in response to an applied electric field.

Each EAP element may be sandwiched between electrodes. The electrodes may be stretchable so that they follow the deformation of the EAP material. Materials suitable for the electrodes should be ultrasound-transmissive and include for instance thin metal films, such as gold, copper, or aluminum or organic conductors such as carbon black, carbon nanotubes, graphene, poly-aniline (PANI), poly(3,4-ethylenedioxythiophene) (PEDOT), e.g. poly(3,4-ethylenedioxythiophene) poly(styrenesulfonate) (PEDOT:PSS).

If the electrodes are arranged in a non-symmetric configuration, the imposed voltage can induce all kinds of deformations such as twisting, rolling, torsioning, turning, and non-symmetric bending deformation.

As discussed above, embodiments of the invention make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A wirelessly controllable device (20), comprising:

   a plurality of actuator elements (22), each actuator element comprising a responsive material, deformable in response to an electrical stimulus for activating the actuator element, and each actuator element being electrically connected with a respective receiver circuit (24) for electrically supplying the actuator element with the electrical stimulus, the receiver circuit including an inductive coil (26) for receiving electrical energy by inductive coupling, and a capacitor (27) for tuning, in combination with the inductive coil, a resonance frequency of the receiver circuit, and wherein each receiver circuit is tuned with a different resonance frequency, to thereby enable selective activation of the actuator elements through application of a magnetic field containing frequency components matching selected of said resonance frequencies.

2. A wirelessly controllable device (20) as claimed in claim 1, wherein each actuator element (22) comprises an actuator member comprising the responsive material and an electrode arrangement arranged for electrically stimulating the actuator member.

3. A wirelessly controllable device (20) as claimed in claim 1 or 2, wherein the wirelessly controllable device (20) is an implantable device.

4. A wirelessly controllable device (20) as claimed in any preceding claim, wherein the responsive material is an electroactive material, and preferably an electroactive polymer.

5. A system (30), comprising:

   a wirelessly controllable device (20) as claimed in any of claims 1 to 4; and
   a control unit (28), comprising:

   a transmitter coil arrangement (32) operable to generate a magnetic field for supplying electrical energy to the receiver circuits (24) of the wirelessly controllable device (20) through inductive coupling, and
   a controller (36), operatively coupled with the transmitter coil arrangement (32), and adapted to effect selective activation of the actuator elements (22) by controlling a frequency spectrum of the magnetic field to include frequency components selectively matching selected of said resonance frequencies of the receiver circuits.

6. A system (30) as claimed in claim 5, wherein the controller (36) is adapted to control the transmitter coil arrangement (32) to generate a magnetic field having a single frequency component which varies over time between different of the resonance frequencies to thereby realize sequential activation of different of the actuator elements (22).

7. A system (30) as claimed in claim 5, wherein the controller (36) is adapted to control the transmitter coil arrangement (32) to generate a magnetic field comprising multiple superposed frequency components matching different of the resonance frequencies, to thereby realize simultaneous activation of different of the actuator elements (22).

**8.** A system (30) as claimed in any of claims 5 to 7, wherein the transmitter coil arrangement (32) comprises a plurality of individual transmitter coils, each controlled by the controller (36) to generate a respective magnetic field of a different respective frequency, matching a resonance frequency of a respective one of the receiver circuits (24).

**9.** A system (30) as claimed in claim 8, wherein said individual transmitter coils are controlled to generate respective magnetic fields simultaneously with one another to thereby realize simultaneous activation of a plurality of the actuator elements (22).

**10.** A system (30) as claimed in claim 8 or 9, wherein said plurality of individual transmitter coils are arranged in planar formation.

**11.** A system (30) as claimed in any of claims 5 to 10, wherein the controller (36) is adapted to control the transmitter coil arrangement (32) to generate a magnetic field comprising a repeating pattern of pulsed wave-packets, each comprising two or more superposed frequency components matching resonance frequencies of two or more of the receiver circuits (24).

**12.** A system (30) as claimed in any of claims 5 to 11, wherein the controller (36) is adapted to control an amplitude of one or more of the frequency components of the generated magnetic field, to thereby control an actuation displacement of the corresponding activated actuator element (22).

**13.** A system (30) as claimed in any of claims 5 to 12, wherein the controller (36) is adapted to control a degree of actuation displacement of a selectively activated actuator element (22) by controlling a degree of correspondence between a matched frequency component of the magnetic field and the resonance frequency of the receiver circuit (24) supplying the actuator element.

**14.** A system (30) as claimed in any of claims 5 to 13, wherein the controller (36) is adapted to control the transmitter coil arrangement (32) to generate a pulsed field, and wherein the controller is adapted to vary an amplitude displacement of one or more activated actuator elements (22) by varying a duty cycle of at least corresponding matched frequency components of the magnetic field.

**15.** A method of wirelessly controlling a device (20), the device comprising a plurality of actuator elements (22), each actuator element comprising a responsive material deformable in response to an electrical stimulus, and each element being electrically supplied by a respective receiver circuit (24) for supplying electrical stimuli for activating the element, wherein each receiver circuit includes

an inductive coil (26) for receiving electrical energy by inductive coupling, and
a capacitor (27) for tuning, in combination with the inductive coil, a resonance frequency of the circuit,

wherein each receiver circuit is tuned to a different resonance frequency, and
wherein the method comprises:

applying a magnetic field to the device having a frequency spectrum controlled to include frequency components selectively matching one or more of the resonant frequencies of the receiver circuits to thereby realize selective activation of a corresponding one or more of the actuator elements.

FIG. 1

FIG. 2

FIG. 3

FIG. 4

FIG. 5

FIG. 6

Europäisches
Patentamt

European
Patent Office

Office européen
des brevets

**EUROPEAN SEARCH REPORT**

Application Number

EP 17 19 4012

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/015918 A1 (LIU YIMING [US] ET AL) 21 January 2010 (2010-01-21) | 1-5, 7-10,15 | INV. H02J50/40 |
| A | * paragraph [0058] - paragraph [0085] * <br> * paragraph [0110] * | 6,11-14 | H01L41/04 H01L41/193 H02J50/12 |
| | ----- | | |
| A | WO 2017/036695 A1 (KONINKLIJKE PHILIPS NV [NL]) 9 March 2017 (2017-03-09) * page 8, line 26 - page 11, line 22; figures 1-4 * | 1-15 | |
| | ----- | | |

TECHNICAL FIELDS
SEARCHED      (IPC)

H02J
H01L

The present search report has been drawn up for all claims

| Place of search | Date of completion of the search | Examiner |
|---|---|---|
| Munich | 19 February 2018 | Telega, Pawel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

17

## ANNEX TO THE EUROPEAN SEARCH REPORT
## ON EUROPEAN PATENT APPLICATION NO.

EP 17 19 4012

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2018

| Patent document cited in search report | Publication date | Patent family member(s) | Publication date |
|---|---|---|---|
| US 2010015918 A1 | 21-01-2010 | NONE | |
| WO 2017036695 A1 | 09-03-2017 | NONE | |

EPO FORM P0459

For more details about this annex : see Official Journal of the European Patent Office, No. 12/82